Europäisches Patentamt

(19) European Patent Office

Office européen des brevets

(11) Veröffentlichungsnummer: **0 097 226**
**B1**

(12) **EUROPÄISCHE PATENTSCHRIFT**

(45) Veröffentlichungstag der Patentschrift:
05.08.87

(51) Int. Cl.⁴: **A 61 F 2/38,** F 16 F 9/20,
F 16 F 9/44

(21) Anmeldenummer: 83103218.0

(22) Anmeldetag: 31.03.83

(54) **Hydraulik-Dämpfzylinder und damit aufgebautes künstliches Gelenk.**

(30) Priorität: 18.06.82 DE 3222885

(43) Veröffentlichungstag der Anmeldung:
04.01.84 Patentblatt 84/1

(45) Bekanntmachung des Hinweises auf die Patenterteilung:
05.08.87 Patentblatt 87/32

(84) Benannte Vertragsstaaten:
AT DE FR GB IT NL SE

(56) Entgegenhaltungen:
BE - A - 395 063
CH - A - 307 417
DE - A - 1 766 909
DE - A - 3 028 608
US - A - 3 352 386
US - A - 4 212 087

(73) Patentinhaber: **Otto Bock Orthopädische Industrie
Besitz- und Verwaltungs-Kommandit-gesellschaft,
Industriestrasse, D-3408 Duderstadt 1 (DE)**

(72) Erfinder: **Glabiszewski, Richard, Im Puttfeld 1,
D-3428 Duderstadt 13 (DE)**

(74) Vertreter: **Lins, Edgar, Dipl.-Phys. et al, Patentanwälte
Gramm + Lins Theodor-Heuss-Strasse 2,
D-3300 Braunschweig (DE)**

## Beschreibung

Die Erfindung betrifft einen Hydraulik-Dämpfzylinder zur Dämpfung der Bewegung künstlicher Gelenke mit einer ringförmigen Kammer für die Hydraulikflüssigkeit, die durch eine ringförmige Schulter eines Kolbens in zwei Teilkammern unterteilt ist, mit beiderseits der Schulter angeordneten, die Dämpfung bestimmenden, mit einer innerhalb des Kolbens angeordneten Ausnehmung verbundenen Umströmungsöffnungen, wobei die durch die Umströmungsöffnungen und die Ausnehmung gebildete Flüssigkeitspassage im Querschnitt einstellbar ist.

Bei Dämpfzylindern sind die Teilkammern im allgemeinen axial hintereinander angeordnet. Es ist auch bekannt, zwei Zylinderräume vorzusehen, wobei der Kolben das Volumen der ersten Teilkammer verringern kann und die Hydraulikflüssigkeit über eine Verbindungsleitung in die volumenmässig vergrösserbare zweite Teilkammer drückt. Die erste Teilkammer ist mit einem Kolben versehen, der durch die Hydraulikflüssigkeit gegen die Kraft des Rückstellelements verschiebbar ist. Derartige Hydraulik.Dämpfzylinder benötigen relativ viel Platz. Obwohl die Bewegung von künstlichen Gelenken durch Hydraulik-Dämpfzylinder in vorteilhafter Weise gedämpft werden kann, stösst die Verwirklichung auf erhebliche Schwierigkeiten, da z.B. bei einem Kniegelenk der Hydraulik-Dämpfzylinder wegen seiner Länge sehr weit in das Wadenteil hinunterreicht, wodurch ein sehr nachteiliger Aufbau entsteht. Insbesondere ist es nicht möglich, einen modularen Aufbau eines künstlichen Gelenkes zu verwirklichen, da dieser voraussetzt, dass das Kniegelenk nicht in das Wadenteil hineinragt, sondern für sich auswechselbar ist.

Durch die US-A-3 352 386 ist ein Hydraulik-Dämpfzylinder bekannt, dessen Kolben hohl ausgebildet ist. Beiderseits der ringförmigen Schulter, die eine mit der Hydraulikflüssigkeit gefüllte Kammer in zwei ringförmige Teilkammern unterteilt, sind Umströmungsöffnungen vorgesehen, durch die Hydraulikflüssigkeit von der einen Teilkammer in die andere strömen kann. Die durch die Umströmungsöffnungen und den Innenraum des hohlen Kolbens gebildete Flüssigkeitspassage ist in ihrem Querschnitt mittels einer Schraube einstellbar. Der Innenraum des Kolbens ist hierzu mit einer durch ein Gewinde gebildeten Wandung versehen, die die Verstellschraube führt.

In der BE-A-395 063 ist ein Stossdämpfer für Kraftfahrzeuge beschrieben, bei dem der Drosselkolben durch Federn in seiner relativen Lage zum Kolben gehalten wird und daher beim Auftreten eines Stosses seine relative Lage zum Kolben ändert, wodurch die gewünschte Dämpfungshärte für den Stoss erzielt wird.

Der Erfindung liegt die Aufgabe zugrunde, einen Hydraulik-Dämpfzylinder der eingangs erwähnten Art zu erstellen, der sich auf kleinem Raum erstellen lässt, eine Rückstellung erlaubt und in einem künstlichen Gelenk so platzsparend untergebracht werden kann, dass ein modularer Aufbau möglich ist.

Diese Aufgabe wird erfindungsgemäss dadurch gelöst, dass die Ausnehmung als ringförmige Ausgleichskammer innerhalb des Kolbens ausgebildet ist, dass die Ausgleichskammer durch die Innenwandung des Kolbens und die Aussenwandung eines Drosselkolbens gebildet ist, dessen relative axiale Lage zum Kolben mit Einstellmitteln veränderbar während der Bewegung des künstlichen Gelenks jedoch fest ist und dass der Drosselkolben hohl ausgebildet ist und ein Rückstellelement für die Bewegung des künstlichen Gelenks umfasst.

Der erfindungsgemässe Hydraulik-Dämpfzylinder zeichnet sich dadurch aus, dass die Einstellung des Dämpfungsgrades mit einem im hohlen Kolben axial verschiebbar geführten Drosselkolben erfolgt, der seinerseits wieder hohl ausgebildet ist und dass vorzugsweise durch eine Schraubenfeder gebildete Rückstellelement umfasst. Dadurch wird bei einem sehr kompakten Aufbau zugleich eine gut einstellbare Dämpfung und eine Rückstellung des Dämpfzylinders in Richtung einer Ausgangsstellung erzielt. Die Ausgleichskammer, durch die die von der ersten Teilkammer in die zweite Teilkammer und umgekehrt strömende Hydraulikflüssigkeit fliesst, kann vorzugsweise mit einer schrägen Wandung ausgebildet sein, die zwischen den beiden Bohrungen durch die Veränderung der relativen Lage des Drosselkolbens zum Kolben verschiebbar ist. Dadurch lässt sich der Strömungswiderstand durch die Verbindung zwischen der ersten Teilkammer und der zweiten Teilkammer regulieren.

Zur Verstellung des Drosselkolbens gegenüber dem Kolben kann in einer einfachen Ausführungsform der Drosselkolben mit einer Stirnfläche an einer in dem Kolben angeordneten Verstellschraube anliegen. Die beispielsweise schräg auf die Stirnfläche treffende Schraube verändert durch ihre Drehung die relative Lage von Kolben und Drosselkolben zueinander.

Es ist vorteilhaft, wenn das Gehäuse des Zylinders im Bereich der Teilkammern eine rippenförmige Aussenfläche aufweist. Die Rippen erfüllen die Aufgabe von Kühlrippen, indem sie die Oberfläche des Zylinders vergrössern, über die Wärme an die Umgebung abgegeben werden kann.

In einer besonders bevorzugten Ausführungsform wird die zwischen Kolben und Gehäuse des Zylinders befindliche Teilkammer durch einen in das Gehäuse des Zylinders einschraubbaren Boden abgeschlossen, der an seinem zur Teilkammer zeigenden Ende eine zur Anlage am Kolben bestimmte Dichtung aufweist. Dieser Aufbau ermöglicht eine einfache Montage und Demontage des Dämpfzylinders, da nach Aufschrauben des Bodens der Kolben mit seiner Schulter leicht aus dem Gehäuse des Zylinders herausgezogen werden kann. Dieser Aufbau ermöglicht aber auch eine besonders einfache Füllung des Hydraulik-Dämpfzylinders, die bei bekannten Hydraulikzylindern umständlich und aufwendig ist.

Hierzu weist der Kolben vorzugsweise im ausgezogenen Zustand in Bezug auf den mit dem Innengewinde zur Aufnahme des Bodens versehenen Teils des Gehäuse des Zylinders eine Länge auf, die ein teilweises Aufschrauben des Bodens erlaubt, ohne dass die Dichtung einen dichtenden Kontakt mit dem Kolben hat. Beim weiteren Einschrauben des Bodens bekommt die Dichtung einen dichtenden Kontakt mit dem Kolben und behält diesen während einer weiteren Einschraubbewegung des Kolbens bei. Vorzugsweise kann dann eine Dichtung in der Aussenwand des Bodens vorgesehen sein, die während der Einschraubbewegung einen dichtenden Kontakt zur Innenwand des Gehäuses des Zylinders herstellt, und zwar gleichzeitig mit oder nach der Abdichtung durch die Dichtung zwischen Boden und Kolben. Der Einfüllvorgang gestaltet sich bei dem erfindungsgemässen Hydraulik-Dämpfzylinder äusserst einfach. Beim abgeschraubten Boden wird der Zylinder voll ausgezogen und die Umströmungsöffnung voll geöffnet. Dann wird die Hydraulikflüssigkeit in die Kammer zwischen Kolben und Gehäuse des Zylinders eingefüllt. Die in der Ausgleichskammer befindliche Luft verhindert, dass die Hydraulikflüssigkeit wesentlich in die Ausgleichskammer eindringt.

Es wird soviel Hydraulikflüssigkeit eingefüllt, dass der Flüssigkeitsspiegel oben mit dem Ende des Kolbens abschliesst. Nunmehr wird der Boden von oben in das nach oben zeigende Ende des gegenüber seiner Einbaustellung auf den Kopf gestellten Zylinders eingeschraubt. Die die Kammer abschliessende ringförmige Wandung gerät dabei in den Kontakt mit der Oberfläche der Hydraulikflüssigkeit. Beim weiteren Einschrauben hat die Dichtung noch keinen dichtenden Kontakt mit dem Kolben, so dass die Flüssigkeit nach oben herausgequetscht wird. Danach erhält die Dichtung zwischen Boden und Kolben den dichtenden Kontakt und auch die Dichtung zwischen Boden und Gehäuse des Zylinders. Beim weiteren Einschrauben wird der dichtende Kontakt beibehalten, wodurch die Hydraulik-Flüssigkeit durch die Umströmungsöffnung in die Ausgleichskammer gedrückt wird. Die darin enthaltene Luft wird dabei komprimiert, bis der Boden vollständig eingeschraubt ist. Die komprimierte Luft wird am in der Einzelstellung unteren Ende der Ausgleichskammer als Luftkissen zusammengedrückt, so dass durch das Aufschrauben des Bodens die Hydraulikflüssigkeit in die Kammer zwischen Kolben und Gehäuse des Zylinders eingefüllt wird und auch die Ausgleichskammer so gefüllt wird, dass der mit der Hydraulikflüssigkeit gefüllte Teil der Kammer mit den zwischen Gehäuse des Zylinders und Kolben befindlichen Teilkammern in Kontakt steht. Das Aufschrauben des Bodens bewirkt daher eine Dosierung der Hydraulikflüssigkeit, eine vollständige Entlüftung der Arbeitskammern sowie eine Teilentlüftung der Ausgleichskammer und eine Füllung der Ausgleichskammer, so dass die darin enthaltene Hydraulikflüssigkeit einen eventuellen Flüssigkeitsverlust in den Arbeitskammern ersetzen kann.

Die Erfindung betrifft weiterhin ein künstliches Gelenk, insbesondere Kniegelenk, mit einer Gelenkachse, um die ein Gelenkoberteil relativ zu einem Gelenkunterteil drehbar ist und mit einem zwischen Gelenkoberteil und Gelenkunterteil angebrachten Hydraulik-Dämpfzylinder. Durch den erfindungsgemässen, kleinbauenden Dämpfzylinder lässt sich ein modulares künstliches Gelenk aufbauen, wenn das Gelenkoberteil und das Gelenkunterteil jeweils einen justierbaren Anschluss für Rohrskeletteile aufweisen und der Dämpfzylinder in dem gabelförmig ausgebildeten Gelenkunterteil gelagert ist.

Das erfindungsgemässe künstliche Gelenk baut nicht grösser als herkömmliche künstliche Gelenke mit einer mechanischen Dämpfung. Daher lässt sich das erfindungsgemässe künstliche Gelenk einfach gegen ein herkömmliches Gelenk austauschen, wobei die Skeletteile völlig unverändert bleiben können. Da bei den bisher bekannten künstlichen Gelenken der Hydraulik-Dämpfzylinder bis in das Wadenteil hineinragte, war ein Austausch bei unveränderten Rohrskeletteilen nicht möglich. Dieser Austausch ist nun durch das erfindungsgemässe künstliche Gelenk ermöglicht worden.

In der erfindungsgemässen Anordnung ist es besonders vorteilhaft, wenn die Achse der Anlenkung des Dämpfzylinders am Gelenkoberteil bei nichtgebeugtem Gelenk unterhalb der Gelenkachse liegt. Bei der erfindungsgemässen Anordnung lässt sich hierdurch eine wirksame Dämpfung der Bewegung des Gelenks in dem Bereich erzielen, der wirkungsvoll gedämpft werden muss. Bei einer stärkeren Beugung des Gelenks entfällt die dämpfende Wirkung des Dämpfzylinders, so dass die Beugung beispielsweise des Kniegelenks ungehindert bis in die Sitzstellung durchgeführt werden kann. Mit dem erfindungsgemässen künstlichen Kniegelenk lässt sich eine wesentlich stärkere Beugung erzielen als bei herkömmlichen Gelenken, da dort die Anlenkung oberhalb der Gelenkachse erfolgt ist, wodurch die stärkere Beugung behindert worden ist.

Es ist besonders vorteilhaft, wenn der Dämpfzylinder einen oberen Ansatz aufweist, der über die Anlenkung hinausragt und mit seinem Ende an dem Gelenkoberteil befestigt ist. Die Anlenkung an dem Gelenkoberteil ist also in die Anordnung des Dämpfzylinders integriert. Lediglich der über die Anlenkung hinausragende Ansatz wird an dem Gelenkoberteil befestigt. Hierdurch lässt sich eine Vereinfachung der Montage erreichen, da die gesamte Dämpfanordnung lediglich an wenigen Schrauben befestigbar ist und somit leicht ausgewechselt werden kann, wenn beispielsweise der Dämpfzylinder Hydraulikflüssigkeit verloren haben sollte und seine Dämpffunktion nicht mehr in dem gewünschten Masse ausübt.

Der besseren Auswechselbarkeit dient auch die drehbare Lagerung des Dämpfzylinders mit seitlichen Lagerzapfen in dem gabelförmigen Gelenkunterteil. Dabei können die Lagerzapfen vorzugsweise in von aussen in Bohrungen des Gelenkunterteils gesteckte Buchsen eingreifen. Auf diese Weise lässt sich der Dämpfzylinder sehr einfach auswechseln, da die Buchsen lediglich aus den Bohrungen entfernt werden müssen. Dies ist besonders einfach möglich, wenn die Buchsen durch Federn gegen ein Herausfallen gesichert sind. Es sind dann lediglich die Federn zu lockern, um die Buchsen aus den Bohrungen herausnehmen zu können und den Dämpfzylinder freizulegen. Wenn Lagerzapfen und Buchsen konisch ausgebildet sind, wird zusammen mit der gefederten Lagerung ein Verkanten des Dämpfzylinders wirksam verhindert. Es findet eine axiale Selbsteinstellung durch die konische Lagerung statt, so dass eine sehr sichere Funktion des Dämpfzylinders gewährleistet ist.

Zur Schonung des Dämpfzylinders ist es vorteilhaft, wenn Anschläge vorgesehen sind, die die relative Bewegung zwischen Gelenkoberteil und Gelenkunterteil begrenzen, bevor der Kolben des Hydraulikzylinders gegen Wandungen des Zylinders stösst. Es hat sich nämlich herausgestellt, dass bei einer Ausnutzung der Anschläge des Hydraulik-Dämpfzylinders zur Begrenzung der Bewegung des künstlichen Gelenks der Zylinder erheblichen Belastungen unterworfen ist und leicht beschädigt wird, so dass die Funktion des künstlichen Gelenks schnell beeinträchtigt wird.

Die Erfindung soll im folgenden anhand eines in der Zeichnung dargestellten Ausführungsbeispiels näher erläutert werden. Es zeigen:

Figur 1 – eine Ansicht des Kniegelenkes von vorn .

Figur 2 – eine Ansicht des Kniegelenkes von der Seite mit teilweiser Schnittdarstellung

Figur 3 – eine Ansicht des Kniegelenkes aus Figur 2 in gebeugter Stellung

Figur 4 – eine Ansicht eines Dämpfzylinders

Figur 5 – eine Schnittdarstellung des Dämpfzylinders ohne oberen Ansatz mit dem Kolben in eingefahrener Stellung

Figur 6 – eine Schnittdarstellung gemäss Figur 5 mit dem Kolben in ausgefahrener Stellung

Figur 7 – eine Schnittdarstellung analog der Darstellung aus Figur 6 nach der Befüllung des Zylinders mit teilweise aufgeschraubtem Boden

Figur 8 – eine Schnittdarstellung gemäss Figur 7 mit voll eingeschraubtem Boden (Ende des Füllvorganges)

Figur 9 – eine Schnittdarstellung des gemäss Figur 7 und 8 befüllten Hydraulikzylinders in seiner Arbeitsstellung mit eingefahrenem Kolben.

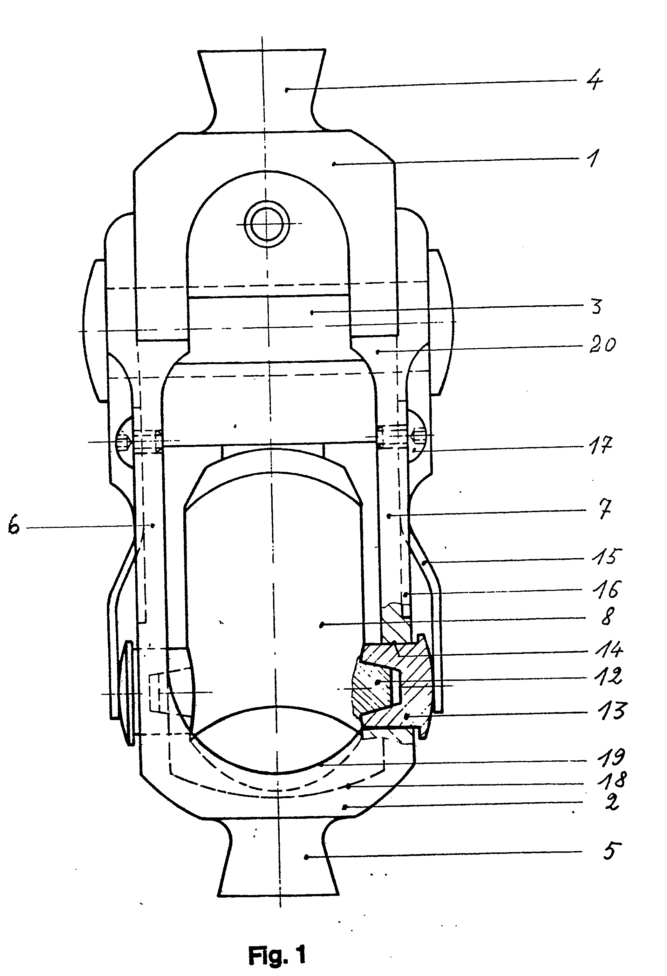

Die Figuren 1 bis 3 zeigen ein künstliches Kniegelenk, das aus einem Gelenkoberteil 1 und einem Gelenkunterteil 2 besteht, die durch eine Gelenkachse 3 miteinander verbunden sind.

Das Gelenkoberteil 1 weist einen justierbaren Anschluss 4 und das Gelenkunterteil 2 einen justierbaren Anschluss 5 auf. An das Gelenkoberteil 1 ist ein Oberschenkel und an das Gelenkunterteil 2 ein Wadenteil in Form von Rohrskeletteilen anschliessbar.

Das Gelenkunterteil 2 ist gabelförmig ausgebildet und besitzt zwei parallel zueinanderlaufende Wandungen 6, 7, zwischen denen ein Hydraulik-Dämpfzylinder 8 angeordnet ist.

Der Hydraulik-Dämpfzylinder ist am Gelenkoberteil 1 mit einem Ansatz 9 befestigt, durch den eine Schraube 10 in das Gelenkoberteil 1 geschraubt ist. Der Hydraulik-Dämpfzylinder 8 ist über ein Drehgelenk 11 mit dem Ansatz 9 verbunden. Das Drehgelenk 11 stellt die Anlenkung des Hydraulik-Dämpfzylinders an dem Gelenkoberteil 1 dar. Die Lagerung des Hydraulik-Dämpfzylinders 8 in dem Gelenkunterteil 2 ist über zu den Seitenwänden 6, 7 zeigende Lagerzapfen 12 erfolgt, die von Buchsen 13 umschlossen sind, die ihrerseits von aussen in Bohrungen 14 in den Wänden 6, 7 gesteckt sind. Die Aussenwände der Lagerzapfen 12 sowie die Innenwände der Buchsen 13 sind konisch ausgebildet, so dass beim Aufstecken der Buchsen 13 auf die Lagerzapfen 12 eine automatische Zentrierung des Hydraulik-Dämpfzylinders 8 in dem Gelenkunterteil 2 erfolgt. Die Buchsen 13 sind jeweils durch einen federnden Bügel 15 gehalten, der sich auf einer Ausnehmung 16 in den Wänden 6, 7 heraus über die Buchsen 13 erhebt und an ihnen federnd anliegt. In der Ausnehmung 16 ist das dort befindliche Teil des Bügels 15 durch eine Schraube 17 befestigt.

Sowohl der Boden 18 des gabelförmigen Gelenkunterteils 2 als auch der Boden 19 des Hydraulik-Dämpfzylinders 8 sind gewölbt, so dass sich der Hydraulik-Dämpfzylinder 8 um die Lagerzapfen 12 drehbar bewegen kann.

Im aufrechtstehenden Zustand des Kniegelenks liegen Anschlagleisten 20 von Gelenkoberteil 1 und Gelenkunterteil 2 aufeinander und begrenzen die Aufrichtbewegung des künstlichen Kniegelenks.

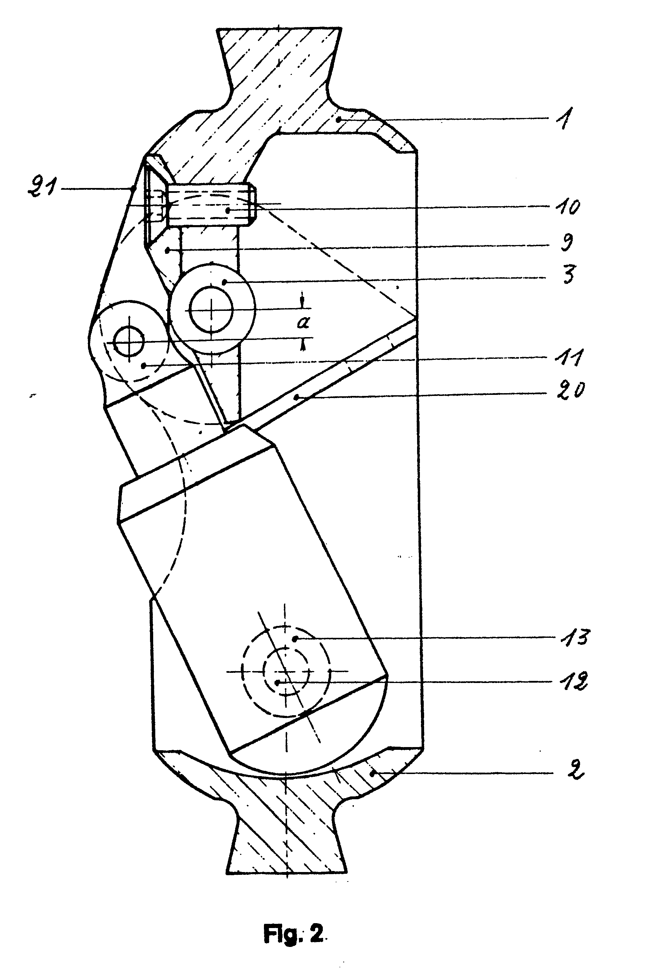

Figur 2 lässt erkennen, dass im aufrechten Zustand des Kniegelenks der Mittelpunkt der Anlenkung 11 um ein Stück a tiefer liegt als der Mittelpunkt der Gelenkachse 3.

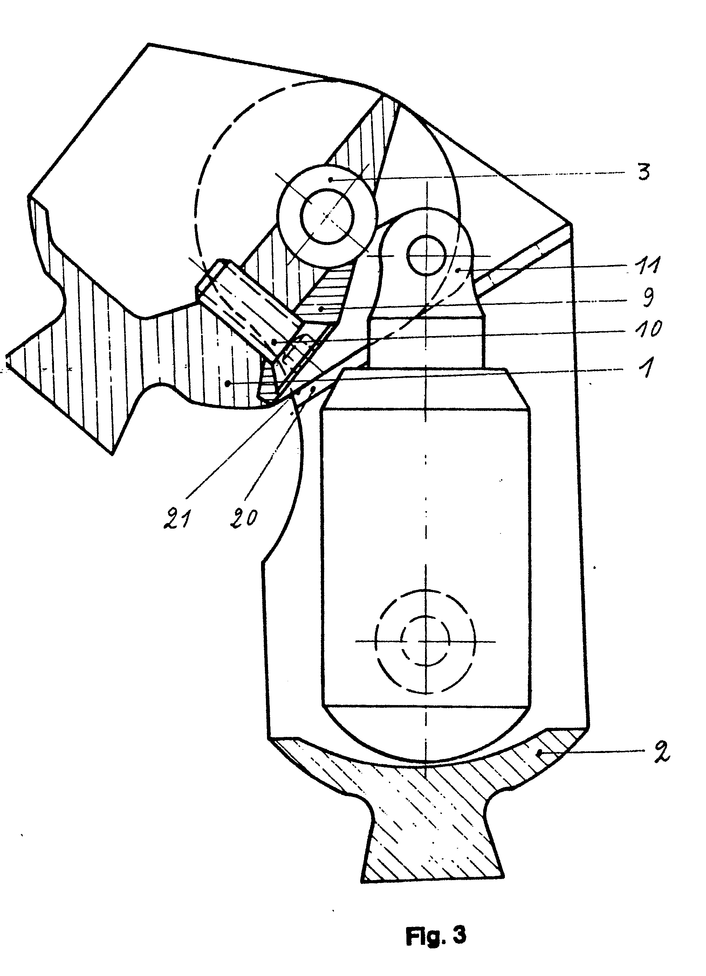

Figur 3 zeigt das Kniegelenk aus Figur 2 im gebeugten Zustand, d.h. in der Sitzstellung. Die Beugebewegung wird wiederum durch die Anschlagleiste 20 des Gelenkunterteils 2 begrenzt, mit der eine Kante 21 auf der Rückseite des Gelenkoberteils 1 zusammenwirkt. Die Neigung der Kante 21 ist so ausgebildet, dass sie flächig auf der Anschlagleiste 20 des Gelenkunterteils 2 aufliegt. Durch die niedrigere Anordnung der Anlenkung 11 gegenüber der Gelenkachse 3 lässt sich eine wesentlich stärkere Biegung des künstlichen Gelenks realisieren als bei herkömmlichen künstlichen Gelenken.

Beim Übergang von der gestreckten Stellung des künstlichen Gelenks in die gebeugte Endstellung aus Figur 3 wird der Hydraulik-Dämpfzylinder durch die Bewegung der Anlenkung 11 auf einer Kreisbahn zunächst zusammengedrückt bis zu einem Biegewinkel von ca. 90°. Danach entspannt sich der Hydraulik-Dämpfzylinder 8 wieder etwas, so dass die Rückstellkraft des Hydraulik-Dämpfzylinders 8 das künstliche Gelenk in der in Figur 3 dargestellten Endstellung hält. Das frühe Unwirksamwerden der Gegenkraft des Hydraulik-Dämpfzylinders 8 ist eine Folge der niedrigen Anordnung der Anlenkung 11 relativ zur Gelenkachse 3.

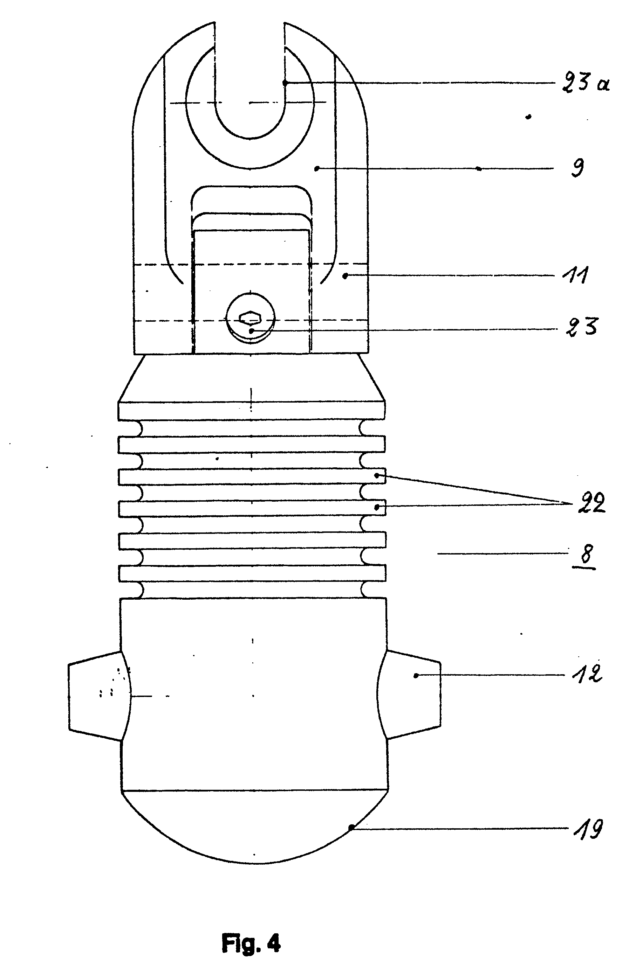

Figur 4 zeigt eine Ansicht eines Hydraulikzylinders 8 im ausgebauten Zustand. An den zylindrischen Grundkörper sind die beiden Lagerzapfen 12 angeformt. Der Grundkörper wird abgeschlossen durch den konvex gewölbten Boden 19. Oberhalb der Lagerzapfen 12 ist die Aussenwandung mit Rippen 22 versehen, die die Oberfläche des Gehäuses vergrössern und einer verstärkten Wärmeabgabe an die Umgebung dienen. Figur 4 lässt das Drehgelenk 11 erkennen, unterhalb dessen sich eine Einstell-Madenschraube 23 befindet, deren Funktion unten näher erläutert wird. Jenseits des Drehgelenks 11 schliesst sich der Ansatz 9 an, der eine Ausnehmung 23a für die Aufnahme der Schraube 10 aufweist.

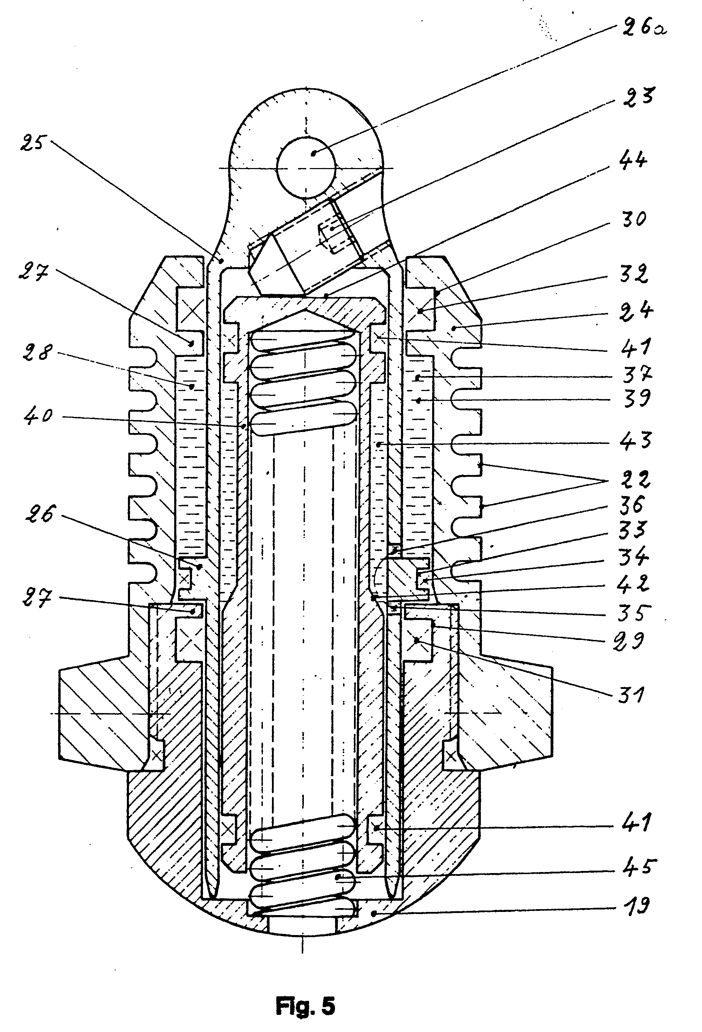

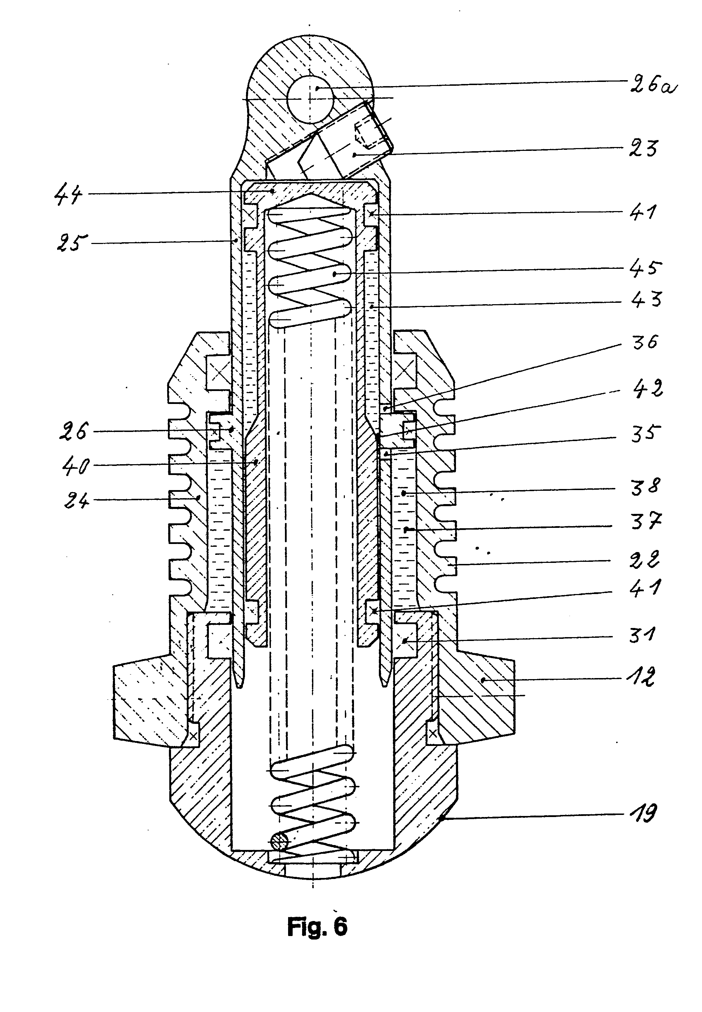

Die Figuren 5 und 6 verdeutlichen den inneren Aufbau des Hydraulik-Dämpfzylinders 8. Das zylindrische Gehäuse 24 besteht aus zwei Teilen, die ineinander verschraubt sind. In dem zylindrischen Gehäuse ist ein Kolben 25 geführt, der mit einer Öse 26a zur Aufnahme einer Drehachse des Drehgelenks 11 verbunden ist. Der Kolben 25 ist hohl ausgeführt. Er weist eine ringförmige Schulter 26 auf, die in einer zwischen zwei Stegen 27 in der Wandung des zylindrischen Gehäuses 24 gebildeten ringförmigen Kammer 28 verschiebbar ist. Figur 5 zeigt den Kolben im eingeschobenen Zustand, Figur 6 den Kolben 25 im ausgezogenen Zustand. Der Kolben 25 ist gegenüber der Kammer 28 mittels zweier in ringförmigen Nuten 29, 30 eingelegten O-Ringen 31, 32 abgedichtet. Die Aussenfläche der Schulter 26 weist ebenfalls eine Nut 33 auf, in die ein weiterer O-Ring 34 eingelegt ist. Beiderseits der Schulter 26 weist der Kolben 25 zwei Umströmungsbohrungen 35, 36 auf, durch die in der Kammer 28 befindliche Hydraulikflüssigkeit 37 in das Innere des Kolbens 25 strömen kann.

Innerhalb der Kammer 28 wird zwischen dem zum Zylinderboden 19 zeigenden Steg 27 und der Schulter 26 eine erste Teilkammer 38 gebildet (Figur 6), während zwischen dem zum Zylinderkopf zeigenden Steg 27 und der Schulter 26 eine zweite Teilkammer 39 besteht. Beim Eindrücken des Kolbens 25 von der ausgezogenen Stellung (Figur 6) in die eingefahrene Stellung (Figur 5) verkleinert sich die erste Teilkammer 38 kontinuierlich, während sich im gleichen Masse die zweite Teilkammer 39 vergrössert.

In dem hohlen Innenraum des Kolbens 25 ist ein weiterer Kolben als Drosselkolben 40 geführt. Der Drosselkolben ist mit zwei O-Ringen 41 an seinen Enden gegenüber dem Innern des Kolbens 25 abgedichtet. In Höhe der beiden Umströmungsbohrungen 35, 36 verläuft die Wandung des Drosselkolbens über eine Schräge 42 in eine ringförmige Ausnehmung 43, die eine Ausgleichskammer bildet. Beim Eindrücken des Kolbens 25 zusammem mit dem Drosselkolben 40 aus der ausgezogenen Stellung (Figur 6) in die eingefahrene Stellung (Figur 5) strömt daher die Hydraulikflüssigkeit 37 aus der ersten Teilkammer 38 durch die erste Umströmungsbohrung 35 in die zweite Teilkammer 43 und von dort durch die zweite Umströmungsbohrung 36 in die Aufnahmekammer 39.

Durch eine Verstellung der relativen Lage des Drosselkolbens 40 gegenüber dem Kolben 25 lässt sich die Lage der Schräge 42 vor den Umströmungsbohrungen 35, 36 verändern. Dadurch wird auch der Strömungswiderstand für die Hydraulikflüssigkeit auf dem Weg von der ersten Teilkammer 38 in die zweite Teilkammer 39 und umgekehrt verändert.

Die Einstellung der relativen Lage des Drosselkolbens 40 gegenüber dem Kolben 25 geschieht mit Hilfe der Verstell-Madenschraube 23, die schräg in den Kolben 25 eingeführt ist und mit einer zylindrischen Schrägfläche gegen die Stirnwandung 44 des Drosselkolbens 40 drückt. Die Gegenkraft zu der Verstellkraft der Schraube 23 wird durch eine Schraubenfeder 45 erzeugt, die im Boden 19 des Hydraulik-Dämpfzylinders 8 einerseits und an der Innenseite der Stirnwandung 44 des hohlen Drosselkolbens 40 andererseits gelagert ist. Die Schraubenfeder 45 drückt den Kolben 25 zusammen mit dem Drosselkolben 40 aus der eingefahrenen Stellung (Figur 5) in die ausgezogene Stellung (Figur 6) zurück.

Es ist ohne weiteres erkennbar, dass die Unterbringung der ersten Teilkammer 38 und der zweiten Teilkammer 39 in derselben Kammer 28 in der Wandung des zylindrischen Gehäuses 24 eine sehr platzsparende Anordnung erreicht wird. Durch die Ausbildung der Kolben 25, 40 als Hohlkolben lässt sich ebenfalls Platz sparen, die Schraubenfeder 45 als Rückstellelement unterbringen. Die Einstellung der Dämpfkraft des Hydraulik-Dämpfzylinders 8 mit Hilfe der Madenschraube 23 und des Drosselkolbens 40 geschieht ebenfalls ohne zusätzlichen Platzaufwand.

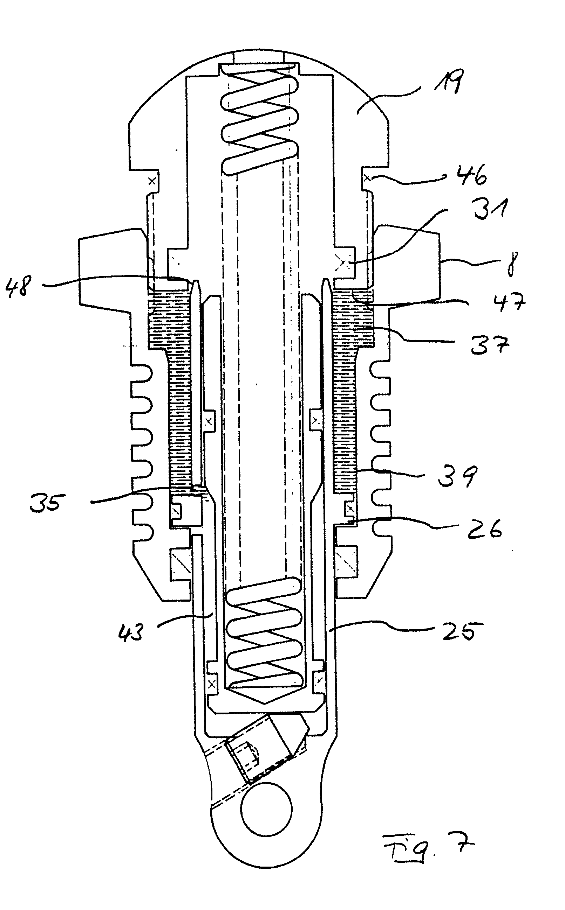

Die Figuren 7 bis 9 verdeutlichen den Einfüllvorgang für die Hydraulikflüssigkeit, der sich bei dem erfindungsgemässen Hydraulik-Dämpfzylinder äusserst einfach gestaltet. Der Zylinder wird in seiner umgedrehten Lage, also mit seinem unteren Ende nach oben, mit Hydraulikflüssigkeit 37 dadurch gefüllt, dass der Boden 19 aufgeschraubt ist und die Hydraulikflüssigkeit 37 in die Teilkammer 39 eingefüllt wird, wobei der Kolben 25 vollständig herausgezogen ist, so dass die Schulter 26 am oberen Anschlag anliegt und die Teilkammer 38 zunächst praktisch auf Null zusammengedrückt ist. Obwohl die Umströmungsöffnung 35 geöffnet ist, strömt praktisch keine Hydraulikflüssigkeit 37 in die Ausgleichskammer 43, da die darin befindliche Luft nicht entweichen kann. Da die Kammer 39 wegen des abgeschraubten Bodens 19 nach oben hin offen ist, lässt sich die Hydraulikflüssigkeit 37 bis zum nach oben zeigenden Ende des Kolbens 25 einfüllen.

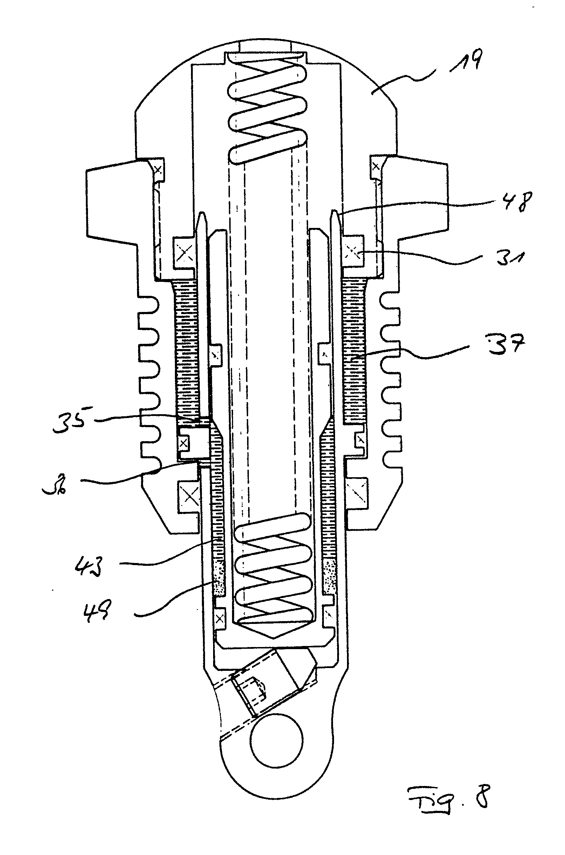

Nunmehr wird der Boden 19 in den Zylinder 8 eingeschraubt. Dabei drückt seine untere, den Abschluss der Teilkammer 39 bildende ringförmige Fläche 47 auf die ringförmige Oberfläche der Hydraulikflüssigkeit 37. Da die Dichtung 31 in dieser Stellung noch keinen dichtenden Kontakt mit dem Kolben 25 hat und eine zwischen Boden 19 und Zylinder 8 vorgesehene Dichtung 46 des Bodens 19 ebenfalls noch nicht abdichtet, entweicht ein Teil der Hydraulikflüssigkeit 37 nach oben aus dem Zylinder 8 heraus. Beim weiteren Einschrauben des Bodens 19 entsteht ein dichtender Kontakt zwischen der Dichtung 31 und dem Kolben 25. Zur Erleichterung dieses Vorganges ist der Kolben an seinem Ende mit einer schräg nach innen laufenden Aussenwand 48 versehen. Durch das weitere Einschrauben des Bodens 19 wird nunmehr die Hydraulikflüssigkeit 37 durch die Umströmungsöffnung 35 in die Ausgleichskammer 43 gedrückt, wobei die darin enthaltene Luft komprimiert wird und ein komprimiertes Luftkissen 49 bildet. Nach dem vollständigen Einschrauben des Bodens 19 ist der Befüllvorgang abgeschlossen. Die Teilkammer 39 ist vollständig luftfrei gefüllt, die Ausgleichskammer 43 ist teilweise gefüllt und weist am in Figur 7 und 8 unteren Ende das komprimierte Luftkissen 49 auf.

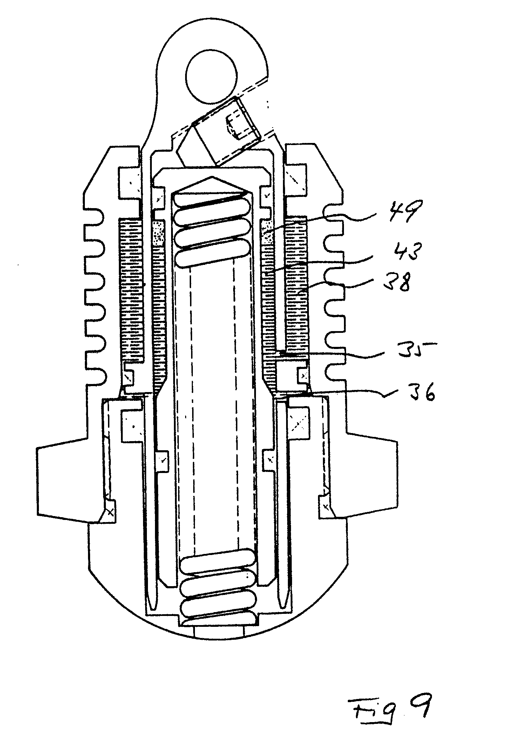

Wie Figur 9 verdeutlicht, gewährleistet diese Befüllung die einwandfreie Funktion des Hydraulik-Dämpfzylinders 8. Durch die Umströmungsöffnungen 35, 36 kann die Hydraulikflüssigkeit zwischen den Teilkammern 38, 39 hin- und herfliessen. Die Ausgleichskammer 43 weist zu den Öffnungen 35, 36 hin eine luftfreie Befüllung auf und ist in der Lage, eventuell entstehende Flüssigkeitsverluste in den Teilkammern 38, 39 zu ersetzen. Das komprimierte Luftpolster 49 hindert dabei nicht, sondern entspannt sich langsam, wenn Flüssigkeit aus der Ausgleichskammer 43 in die Teilkammern, 38, 39 gelangt. Das komprimierte Luftpolster 49 übt daher eine gewisse elastische Nachfüllkraft aus.

**Patentansprüche**

1. Hydraulik-Dämpfzylinder zur Dämpfung der Bewegung künstlicher Gelenke mit einer ringförmigen Kammer (38, 39) für die Hydraulikflüssigkeit (37), die durch eine ringförmige Schulter (26) eines Kolbens (25) in zwei Teilkammern (38 bzw. 39) unterteilt ist, mit beiderseits der Schulter (26) angeordneten, die Dämpfung bestimmenden, mit einer innerhalb des Kolbens (25) angeordneten Ausnehmung verbundenen Umströmungsöffnungen (35, 36), wobei die durch die Umströmungsöffnungen (35, 36) und die Ausnehmung (43) gebildete Flüssigkeitspassage im Querschnitt einstellbar ist, dadurch gekennzeichnet, dass die Ausnehmung als ringförmige Ausgleichskammer (43) innerhalb des Kolbens (25) ausgebildet ist, dass die Ausgleichskammer (43) durch die Innenwandung des Kolbens (25) und die Aussenwandung eines Drosselkolbens (40) gebildet ist, dessen relative axiale Lage zum Kolben mit Einstellmitteln (23) veränderbar während der Bewegung des künstlichen Gelenks jedoch fest ist und dass der Drosselkolben (40) hohl ausgebildet ist und ein Rückstellelement (45) für die Bewegung des künstlichen Gelenks umfasst.

2. Hydraulik-Dämpfzylinder nach Anspruch 1, dadurch gekennzeichnet, dass die Aussenwandung des Drosselkolbens (40) eine die Ausgleichskammer (43) im Bereich der Umströmungsöffnungen (35, 36) begrenzende Schräge (42) aufweist.

3. Hydraulik-Dämpfzylinder nach Anspruch 1 oder 2, dadurch gekennzeichnet, dass das Rückstellelement durch eine Schraubenfeder (45) gebildet ist.

4. Hydraulik-Dämpfzylinder nach einem der Ansprüche 1 bis 3, dadurch gekennzeichnet, dass der Drosselkolben (40) mit einer Stirnfläche (44) an einer in dem Kolben (25) angeordneten Verstellschraube (23) anliegt.

5. Hydraulik-Dämpfzylinder nach einem der Ansprüche 1 bis 4, dadurch gekennzeichnet, dass das Gehäuse (24) des Zylinders im Bereich der Teilkammern (38, 39) eine rippenförmige Aussenfläche (22) aufweist.

6. Hydraulik-Dämpfzylinder nach einem der Ansprüche 1 bis 5, dadurch gekennzeichnet, dass die zwischen Kolben (25) und dem Gehäuse (24) des Zylinders (8) befindliche Teilkammer (38) durch einen in das Gehäuse (24) einschraubbaren Boden (19) abgeschlossen ist, der an seinem zur Teilkammer (38) zeigenden Ende eine zur Anlage am Kolben (25) bestimmte Dichtung (31) aufweist.

7. Hydraulik-Dämpfzylinder nach Anspruch 6, dadurch gekennzeichnet, dass der Kolben (25) im ausgezogenen Zustand in Bezug auf den mit dem Innengewinde zur Aufnahme des Bodens (19) aufweisenden Teils des Gehäuses (24) des Zylinders (8) eine Länge aufweist, die ein teilweises Aufschrauben des Bodens (19) erlaubt, ohne dass die Dichtung (31) einen dichtenden Kontakt mit dem Kolben (25) hat.

8. Hydraulik-Dämpfzylinder nach Anspruch 6 oder 7, dadurch gekennzeichnet, dass die Dichtung (31) beim Einschrauben des Bodens (19) einen dichtenden Kontakt mit dem Kolben (25) bekommt und diesen während einer weiteren Einschraubbewegung des Bodens (19) beibehält.

9. Hydraulik-Dämpfzylinder nach Anspruch 8, dadurch gekennzeichnet, dass eine Dichtung (46) in der Aussenwand des Bodens (19) vorgesehen ist, die während der Einschraubbgewegung einen dichtenden Kontakt zur Innenwand des Gehäuses (24) des Zylinders (8) herstellt, und zwar gleichzeitig mit oder nach der Abdichtung durch die Dichtung (31).

10. Hydraulik-Dämpfzylinder nach einem der Ansprüche 6 bis 9, dadurch gekennzeichnet, dass das zum Boden (19) zeigende Ende des Kolbens (25) eine schräg nach innen auslaufende Aussenwand (48) aufweist.

11. Künstliches Gelenk, insbesondere Kniegelenk, mit einer Gelenkachse (3), um die ein Gelenkoberteil (1) relativ zu einem Gelenkunterteil (2) drehbar ist und mit einem zwischen Gelenkoberteil (1) und Gelenkunterteil (2) angebrachten Hydraulik-Dämpfzylinder nach einem der Ansprüche 1 bis 5, wobei das Gelenkoberteil (1) und das Gelenkunterteil (2) jeweils einen justierbaren Anschluss (4, 5) für Rohrskeletteile aufweisen, dadurch gekennzeichnet, dass der Hydraulik-Dämpfzylinder (8) in dem gabelförmig ausgebildeten Gelenkunterteil gelagert ist.

12. Künstliches Gelenk nach Anspruch 11, dadurch gekennzeichnet, dass die Achse der Anlenkung (11) des Hydraulik-Dämpfzylinders (8) am Gelenkoberteil (1) bei nichtgebeugtem Gelenk unterhalb der Gelenkachse (3) liegt.

13. Künstliches Gelenk nach Anspruch 11 oder 12, dadurch gekennzeichnet, dass der Hydraulik-Dämpfzylinder (8) einen oberen Ansatz aufweist, der über die Anlenkung (11) hinausragt und mit seinem Ende an dem Gelenkoberteil (1) befestigt ist.

14. Künstliches Gelenk nach einem der Ansprüche 11 bis 13, dadurch gekennzeichnet, dass der Hydraulik-Dämpfzylinder (8) mit seitlichen Lagerzapfen (12) in dem gabelförmigen Gelenkoberteil (2) drehbar gelagert ist.

15. Künstliches Gelenk nach Anspruch 14, dadurch gekennzeichnet, dass die Lagerzapfen (12) von aussen in Bohrungen (14) des Gelenkunterteils (2) gesteckte Buchsen (13) eingreifen.

16. Künstliches Gelenk nach Anspruch 15, dadurch gekennzeichnet, dass die Buchsen (13) durch Federn (15) gegen ein Herausfallen gesichert sind.

17. Künstliches Gelenk nach Anspruch 15 oder 16, dadurch gekennzeichnet, dass die Lagerzapfen (12) und die Buchsen (13) konisch ausgebildet sind.

18. Künstliches Gelenk nach einem der Ansprüche 15 bis 17, dadurch gekennzeichnet, dass Anschläge (20, 21) vorgesehen sind, die die relative Bewegung zwischen Gelenkoberteil (1) und Gelenkunterteil (2) begrenzen, bevor der Kolben (25, 26) des Hydraulik-Dämpfzylinders (8) gegen Wandungen (27) des Gehäuses (24) des Hydraulik-Dämpfzylinders (24) stösst.

**Claims**

1. Hydraulic damping cylinder for damping the movement of artificial joints with an annular chamber (38, 39) for the hydraulic liquid (37), siad chamber is divided into two partial chambers (38 and 39, resp.) by means of an annular shoulder (26) of piston (25), with flow openings (35, 36) provided at opposite sides of said shoulder (26) determining the damping action and being connected with in a recess positioned within the piston (25), wherein the fluid passage formed by said flow openings (35, 36) and said recess (43) is adjustable in cross section, characterized in that said recess is formed as an annular compensating chamber (43) within the piston (25), that said compensating chamber (43) is formed by the inner wall of said piston (25) and the outer wall of a throttle piston (40), the axial position of which relative to said piston being variable by adjusting means is, however, fixed during the movement of the artificial joint and that the throttle piston (40) is hollow and embraces a restoring element (45) for the movement of the artificial joint.

2. Hydraulic damping cylinder according to claim 1, characterized in that the outer wall of the throttle piston (40) comprises a slope (42) limiting the compensating chamber (43) in the range of the flow openings (35, 36).

3. Hydraulic damping cylinder according to claim 1 or 2, characterized in that the restoring element is formed by a coil spring (45).

4. Hydraulic damping cylinder according to claims 1 to 3, characterized in that the throttle piston (40) abuts an adjusting screw (23) positioned in the piston (25) with a front surface (44).

5. Hydraulic damping cylinder according to one of the claims 1 to 4, characterized in that the housing (24) of the cylinder comprises a rib-formed outer surface (22) in the range of the partial chambers (38, 39).

6. Hydraulic damping cylinder according to one of the claims 1 to 5, characterized in that said partial chamber (38) arranged between piston (25) and housing (24) of said cylinder (8) is closed by a bottom (19) screwable into the housing (24), said bottom comprising a sealing element (31) at the end directed to the partial chamber (38) and said sealing element being fixed for engaging the piston (25).

7. Hydraulic damping cylinder according to claim 6, characterized in that said piston (25) being in fully pulled out condition has a length in relation to the part of the housing of the cylinder (8) with the internal thread for inserting the bottom (19) which allows a partial screwing of the bottom (19), during which the sealing element (31) has got no sealing contact with the piston (25).

8. Hydraulic damping cylinder according to claim 6 or 7, characterized in that the sealing element (31) comes into a sealing contact with the piston (25) during the screwing of the bottom (19) and keeps said contact during a further screwing movement of the bottom (19).

9. Hydraulic damping cylinder according to claim 8, characterized in that a sealing element (46) is provided in the outer wall of the bottom (19), said selaing element makes a sealing contact to the inner wall of the housing of the cylinder (8) during the screwing movement, namely simultaneously with or after the sealing by sealing element (31).

10. Hydraulic damping cylinder according to one of claims 6 to 9, characterized in that the end of the pistoh (25) directed to the bottom (19) comprises an outer wall (48) sloping inwardly.

11. Artificial joint, especially knee joint, with a hinge axle (3), around which an upper joint part (1) is movable relative to a lower joint part (2) and with a hydraulic damping cylinder according to one of claims 1 to 5 which is positioned between the upper joint part (1) and the lower joint part (2), wherein the upper joint part (1) and the lower joint part (2) each comprise an adjustable connection (4, 5) for tubular skeleton structures characterized in that the hydraulic damping cylinder (8) is mounted in the forkedshaped lower joint part.

12. Artificial joint according to claim 11, characterized in that the axis of the pivot (11) linking the hydraulic damping cylinder (8) at the upper joint part (1) lies below the hinge axle (3) when the joint is not bended.

13. Artificial joint according to claim 11 or 12, characterized in that the hydraulic damping cylinder (8) comprises an upper projection extending over the pivot (11) and being attached with its end at the upper joint part (1).

14. Artificial joint according to one of the claims 11 to 13, characterized in that the hydraulic damping cylinder (8) is pivotably in the forked-shaped upper joint part (2) by means of lateral pivot pins (12).

15. Artificial joint according to claim 14, characterized in that the pivot pins (12) engage with bearings (13) inserted from outside into bores (14) of the lower joint part (2).

16. Artificial joint according to claim 15, characterized in that the bearings (13) are secured against dropping out by springs (15).

17. Artificial joint according to claim 15 or 16, characterized in that the pivot pins (12) and the bearings (13) are formed conically.

18. Artificial joint according to one of the claims 15 to 17, characterized in that stops (20, 21) are provided which limit the relative movement between the upper joint part (1) and the lower joint part (2) before the piston (25, 26) of the hydraulic damping cylinder abuts the walls (27) of the housing (24) of the hydraulic damping cylinder (24).

**Revendications**

1. Cylindre amortisseur hydraulique pour amortir le mouvement d'articulations artificielles, comportant une chambre annulaire (38, 39) pour le fluide hydraulique (37), qui est subdivisée par un épaulement annulaire (26) d'un piston (25) en deux chambres partielles (38 ou 39), tandis que des orifices de contournement (35, 36) déterminant l'amortissement sont disposés de part et d'autre de l'épaulement (26) et reliés à une cavité aménagée à l'intérieur du piston (25), le trajet du fluide, formé par les orifices de contournement (35, 36) et la cavité (43), étant réglable en section transversale, caractérisé en ce que la cavité est conformée en chambre annulaire de compensation (43) à l'intérieur du piston (25), en ce que la chambre de compensation (43) est constituée par la paroi interne du piston (25) et la paroi externe d'un piston d'étranglement (40) dont la position axiale par rapport au piston (25) est réglable par des moyens de réglage (23) mais est fixe pendant les mouvements de l'articulation artificielle, et en ce que le piston d'étranglement (40) est creux et entoure un élément de rappel (45) pour le mouvement de l'articulation artificielle.

2. Cylindre amortisseur hydraulique selon la revendication 1, caractérisé en ce que la paroi extérieure du piston d'étranglement (40) présente un biseau (42) limitant la chambre de compensation (43) au voisinage des orifices de contournement (35, 36).

3. Cylindre amortisseur hydraulique selon la revendication 1 ou 2, caractérisé en ce que l'élément de rappel est constitué par un ressort hélicoïdal (45).

4. Cylindre amortisseur selon l'une des revendications 1 à 3, caractérisé en ce que le piston d'étranglement (40) s'appuie par une face frontale (44) sur une vis de réglage (23) disposée dans le piston (25).

5. Cylindre amortisseur hydraulique selon l'une des revendications 1 à 4, caractérisé en ce que le carter (24) du cylindre présente dans la zone des chambres partielles (38, 39) une surface externe (22) nervurée.

6. Cylindre amortisseur hydraulique selon l'une des revendications 1 à 5, caractérisé en ce que la chambre partielle (38) se trouvant entre le piston (25) et le carter (24) du cylindre (8) est fermée par un fond (19) vissable dans le carter (24), ledit fond présentant, à son extrémité dirigée vers la chambre partielle (38), un joint (31) destiné à prendre appui sur le piston (25).

7. Cylindre amortisseur hydraulique selon la revendication 6, caractérisé en ce que le piston (25) à l'état étiré présente, par rapport à la partie du carter (24) du cylindre (8) comportant le filetage intérieur pour recevoir le fond (19), une longueur qui permet de visser partiellement le fond (19) sans que le joint (31) ait un contact étanche avec le piston (25).

8. Cylindre amortisseur hydraulique selon la revendication 6 ou 7, caractérisé en ce que, lors du vissage du fond (19), le joint (31) établit un contact étanche avec le piston (25) et conserve ce contact pendant la suite du mouvement de vissage du fond (19).

9. Cylindre amortisseur hydraulique selon la revendication 8, caractérisé en ce qu'il est prévu, dans la paroi externe du fond (19), un joint (46) qui établit pendant le mouvement de vissage un contact étanche avec la paroi interne du carter (24) du cylindre (8), et cela en même temps ou après que le joint (31) ait établi l'étanchéïté.

10. Cylindre amortisseur hydraulique selon l'une des revendications 6 à 9, caractérisé en ce que l'extrémité du piston (25) dirigée vers le fond (19) présente une paroi externe (48) s'étendant obliquement vers l'intérieur.

11. Articulation artificielle, en particulier articulation du genou, comprenant un axe d'articulation (3) autour duquel une partie supérieure (1) de l'articulation est rotative par rapport à une partie inférieure (2) de l'articulation, et un cylindre amortisseur hydraulique conforme à l'une des revendications 1 à 5 installé entre les parties supérieure (1) et inférieure (2) de l'articulation, la partie supérieure (1) et la partie inférieure (2) de l'articulation présentant chacune un raccord ajustable (4, 5) pour des organes de squelette tubulaires, caractérisée en ce que le cylindre amortisseur hydraulique (8) est logé dans la partie inférieure de l'articulation réalisée en forme de fourche.

12. Articulation artificielle selon la revendication 11, caractérisé en ce que l'axe de la charnière (11) entre le cylindre amortisseur hydraulique (8) et la partie supérieure (1) de l'articulation est situé en dessous de l'axe (3) de l'articulation quand celle-ci n'est pas repliée.

13. Articulation artificielle selon la revendication 11 ou 12, caractérisée en ce que le cylindre amortisseur hydraulique (8) comporte une rallonge supérieure qui s'élève au-dessus de la charnière (11) et qui est fixée par son extrémité à la partie supérieure (1) de l'articulation.

14. Articulation artificielle selon l'une des revendications 11 à 13, caractérisée en ce que le cylindre amortisseur hydraulique (8) est monté rotativement par des tourillons latéraux (12) dans la partie inférieure fourchue (2) de l'articulation.

15. Articulation artificielle selon la revendication 14, caractérisée en ce que les tourillons (12) pénètrent dans des coussinets (13) insérés de l'extérieur dans des alésages (14) de la partie inférieure (2) de l'articulation.

16. Articulation artificielle selon la revendication 15, caractérisée en ce que les coussinets (13) sont arrêtés vers l'extérieur par des ressorts (15).

17. Articulation artificielle selon la revendication 15 ou 16, caractérisée en ce que les tourillons (12) et les coussinets (13) ont une conformation conique.

18. Articulation artificielle selon l'une des revendications 15 à 17, caractérisée en ce que des butées (20, 21) sont prévues pour limiter le mouvement relatif entre les parties supérieure (1) et inférieure (2) de l'articulation, avant que le piston (25, 26) du cylindre amortisseur hydraulique (8) heurte des parois (27) du carter (24) du cylindre amortisseur hydraulique.

**Fig. 1**

21 —

1

10

9

3

11

20

13

12

2

**Fig. 2.**

**Fig. 3**

**Fig. 4**

**Fig. 5**

26a

23

44

41

25

45

43

36

42

26

35

40

38

24

37

22

41

31

12

19

**Fig. 6**

Fig. 7

Fig. 8

Fig 9